# EUROPEAN PATENT APPLICATION

(11) **EP 3 443 885 A1**
(43) Date of publication of application: **20.02.2019**
(21) Application number: 17186250.1
(22) Date of filing: 15.08.2017
(51) Int. Cl.: A61B 5/00, A61B 5/0245

(54) **ATTRACTION-ATTACHABLE ELECTRICALLY CONDUCTIVE PAD**

(71) Applicant: King's Metal Fiber Technologies Co., Ltd., Taipei City 10437 (TW)
(72) Inventor: WU, Cheng Han, 10437 Taipei City (TW); CHEN, Reng Sho, 10437 Taipei City (TW); HUANG, Hong Hsu, 10437 Taipei City (TW)
(74) Representative: Cabinet Chaillot

(57) **Abstract**

An attraction-attachable electrically conductive pad includes a conductive film and at least one attaching film. The at least one attaching film is coupled to the conductive film and has a surface on which a plurality of miniature structures in the form of projections are formed such that the conductive film is attachable to a human skin through an attractive force of positive and negative charges between molecules of the projections. As such, in use, the arrangement of being attachable to a human skin through an attractive force of positive and negative charges between molecules of the projections helps prevent positional shifting of the conductive film during a detection operation and helps keep in a state of contacting a body surface, and helps keep comfortable and air ventilating for long term wear in order to achieve effective detection of physiological signals.

## Description

### BACKGROUND OF THE INVENTION

### 1. Field of the Invention

The present invention relates generally to an attraction-attachable electrically conductive pad, and more particularly to an electrically conductive pad that can be held in a condition of being in contact engagement with a surface of a human body to conduct detection without accident or undesired position shifting and achieves comfortableness of being worn so as to be fit to operations of physiological detection.

### 2. Description of Related Art

Recently, high-tech manufacturers have actively engaged in studies of installing electrically conductive pads in fabric or woven and knitted articles to detect physiological signals when worn in order to get aware of the states of physical functionality and also for monitoring and recording.

Such a conductive pad is put in contact with the surface of a human body, but such a conductive pad is fixed in the fabric so that movement or activity of the human body would cause the fabric to move and displacement results and would inevitably cause interruption of signal or abnormal signal for the detection signal generated by the conductive pad that is in operation for detection on the body surface. This, once happening, requires re-positioning of the conductive pad, or would make it necessary for the human body to keep still in order to allow for smooth performance of detection of physiological signals.

Thus, in view of the above problems, those known devices or articles are still defective and are not designs of perfectness, and further improvements are necessary.

### SUMMARY OF THE INVENTION

In view of the above, based on years' experience in developing and making of products in the field, the present inventor, after having devoted himself in careful design and evaluation in respect to the above target, have created the present invention, which is believed a practical and useful creation.

The primary objective of the present invention is to provide an attraction-attachable electrically conductive pad, which helps prevent positional shifting of a conductive film during a detection operation and helps keep in a state of contacting a body surface, and helps keep comfortable for long term wear.

According to the above objective, the present invention provides an attraction-attachable electrically conductive pad, which comprises a conductive film and at least one attaching film, wherein the at least one attaching film is coupled to the conductive film and has a surface on which a plurality of miniature structures in the form of projections are formed such that the conductive film is attachable to a human skin through an attractive force of positive and negative charges between molecules of the projections. As such, in use, the arrangement of being attachable to a human skin through an attractive force of positive and negative charges between molecules of the projections helps prevent positional shifting of the conductive film during a detection operation and helps keep in a state of contacting a body surface, and helps keep comfortable and air ventilating for long term wear in order to achieve a purpose of effectively detecting physiological signals.

For better appreciation and understanding of the purpose, shape, structure, device features, and effectiveness of the present invention for Examiner, embodiments are provided as example, with reference to the attached drawings for clear illustration in the following.

### BRIEF DESCRIPTION OF THE DRAWINGS

FIG. 1 is a perspective view showing an attraction-attachable electrically conductive pad according to the present invention;
FIG. 2 is a cross-sectional view showing the attraction-attachable electrically conductive pad according to the present invention;
FIG. 3 is a perspective view showing an attraction-attachable electrically conductive pad according to another embodiment of the present invention; and
FIG. 4 is a perspective view showing an attraction-attachable electrically conductive pad according to a further embodiment of the present invention.

### DETAILED DESCRIPTION OF THE PREFERRED EMBODIMENTS

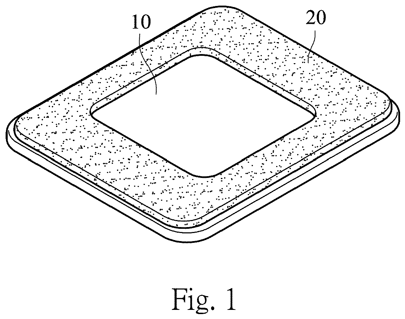

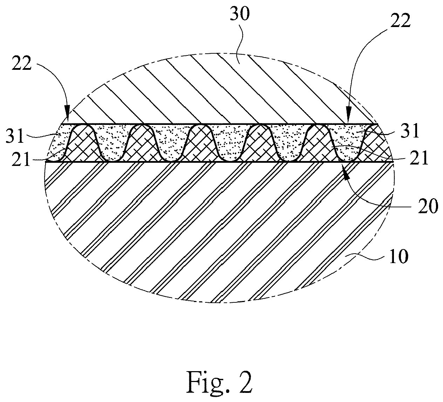

The present invention relates to an attraction-attachable electrically conductive pad. Referring to FIGS. 1 and 2, the attraction-attachable electrically conductive pad of the present invention comprises a conductive film 10 and at least one attaching film 20.

The at least one attaching film 20 is mounted to the conductive film 10 and has a surface on which a plurality of miniature structures, in the form of projections or raised block 21, is formed to allow the conductive film 10 to attach, through an attractive force induced between positive and negative electrical charges of molecules of the projections 21, to a human skin 30.

As an assembly formed with a combination of the above components, in use, the positive-negative charge attractive force induced by the molecules the projections 21 can be used to attach, through attraction, to the human skin 30 and such an arrangement provides a confortable feeing and air ventilation for long term use and also helps prevent undesired positional shift of the conductive film 10 during the use thereof and also helps keep a state of being in contact with the surface of human body, to effectively prevent the performance of detection of physiological signals conducted by the conductive film 10 thereby achieving effectiveness and purpose of accurate performance of physiological signal detection.

Referring back to FIGS. 1 and 2, the positive-negative charge attractive force induced by the molecules of the projections 21 is an effect of Van Der Waals Force.

Referring to FIGS. 1 and 2 again, in a first embodiment of the present invention, the attaching film 20 is coupled to a circumferential area of the conductive film 10 (wherein in the first embodiment, the attaching film 20 is mounted to a circumferential area of an upper surface of the conductive film 10, and alternatively, the conductive film 10 and the attaching film 20 can be set on a common, same, plane (not shown in the drawings) and the attaching film 20 is arranged along a circumferential edge of the conductive film 10), and the coupling is made through adhesive bonding.

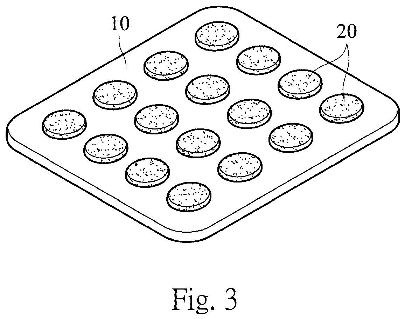

Referring to FIG. 3, a second embodiment of the present invention is shown, in which the attaching film 20 is provided in multiplicity, in the form of a circular or polygonal shape, with the multiple attaching films arranged in a regular manner (such as a regular two-dimensional array) and coupled to a surface of the conductive film 10, wherein the coupling is made through adhesive bonding.

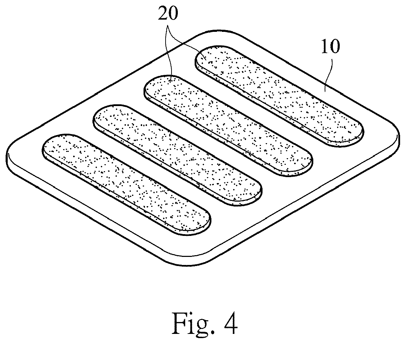

Referring to FIG. 4, a third embodiment of the present invention is shown, in which the attaching film 20 is provided in multiplicity, in the form of elongated strips, with the multiple attaching films arranged in a regular manner (such as a regular one-dimensional array) and coupled to a surface of the conductive film 10, wherein the coupling is made through adhesive bonding.

Referring back to FIGS. 1 and 2, a preferred example of the attraction-attachable electrically conductive pad is applicable to a physiological detection device (not shown in the drawings) to achieve an effect of preventing positional shift of the conductive film 10 during an operation of detection and keeping a state of contact engagement through being fixedly mounted to a surface of a human body to ensure effective detection of physiological signal thereby improving detection performance thereof.

Referring to FIGS. 1 and 2, the conductive film 10 is connected to a signal transmission terminal (not shown in the drawings) and the signal transmission terminal is connected, through conductive wires, to a signal transmitter (not shown in the drawings), wherein the signal transmitter is provided therein with a wireless transmission module (not shown in the drawings) to transmit the physiological signals received thereby and variations thereof in a wireless manner to an electronic device (such as a smart phone, a smart tablet computer, a notebook computer, a desktop computer, and medical equipment) or the cloud for the purpose of easy awareness of the result of detection to provide advanced medical treatment, if necessary.

Referring to FIGS. 1, 2, 3, and 4, the conductive film 10 may be for example one of electrically conductive fabric, an electrode plate, an electrically conductive coating, or an eclectically conductive film of polyurethane (PU). The conductive film 10 is tightly engageable with the skin 30 of a human body to detect surface physiological signals, wherein the physiological signal can be one of body temperature, heartbeat, pulse, and breath.

Referring to FIGS. 1 and 2, the attaching film 20 is electrically conductive and the conductive film 10 is attachable to the human skin 30 with the attractive force induced between positive and negative charges of the molecules of the projections 21 so that moisture 31 from the human skin 30 is allowed to spread into gaps 22 between the projections 21 so that the moisture 31 may get filled in the gaps 22, wherein the moisture 31 that is present in the gaps 22 and minute amounts of metals and minerals contained in the moisture 31 may help improve electrical conductivity of the attaching film 20. Further, the attaching films 20 shown in the embodiments of FIGS. 3 and 4 are also electrically conductive.

The above description provides just preferred embodiments of the present invention and structural features of the present invention are not limited to the description. All the variations and modifications that are easily contemplated by those having ordinary skills in the art within the field of the present invention are considered covered by the scope of the appended claims of the present invention.

## Claims

1. An attraction-attachable electrically conductive pad, **characterized by** comprising:
a conductive film (10); and
at least one attaching film (20), the at least one attaching film (20) being coupled to the conductive film (10) and having a surface on which a plurality of miniature structures in the form of projections (21) are formed such that the conductive film (10) is adapted to attach to a human skin (30) through an attractive force of positive and negative charges between molecules of the projections (21).

2. The attraction-attachable electrically conductive pad as claimed in Claim 1, wherein the attractive force is achieved with an effect of Van Der Waals Force.

3. The attraction-attachable electrically conductive pad as claimed in Claim 1, wherein the attaching film (20) is mounted to a circumferential area of the conductive film (10).

4. The attraction-attachable electrically conductive pad as claimed in Claim 1, wherein the at least one attaching film (20) comprises multiple pieces in the form of circular or polygonal shapes mounted, in a regular arrangement, to a surface of the conductive film (10).

5. The attraction-attachable electrically conductive pad as claimed in Claim 1, wherein the at least one attaching film (20) comprises multiple pieces in the form of elongated strips mounted, in a regular arrangement, to a surface of the conductive film (10).

6. The attraction-attachable electrically conductive pad as claimed in Claim 1, 3, 4, or 5, wherein the attaching film (20) is electrically conductive.

7. The attraction-attachable electrically conductive pad as claimed in Claim 1, 3, 4, or 5, wherein the coupling is achieved with adhesive bonding.

8. The attraction-attachable electrically conductive pad as claimed in Claim 1, wherein the conductive film (10) is further connected to a signal transmission terminal and the signal transmission terminal is connected, through conductive wires, to a signal transmitter, wherein the signal transmitter comprises a wireless transmission module that transmits a signal received thereby.

9. The attraction-attachable electrically conductive pad as claimed in Claim 1 or 8, wherein the conductive film (10) comprises one of electrically conductive fabric, an electrode plate, and an electrically conductive coating film and the conductive film (10) is tightly engageable with a skin (30) of a human body to detect a physiological signal of a surface of the human body, wherein the physiological signal comprises one of body temperature, heartbeat, pulse, and breath.
